(19) Europäisches Patentamt
European Patent Office
Office européen des brevets

(11) EP 2 790 676 B1

(12) **EUROPEAN PATENT SPECIFICATION**

(45) Date of publication and mention
of the grant of the patent:
**10.01.2018 Bulletin 2018/02**

(51) Int Cl.:
*A61K 9/20* (2006.01)       *A61K 31/451* (2006.01)
*A61K 31/445* (2006.01)      *A61K 31/80* (2006.01)

(21) Application number: **12819146.7**

(22) Date of filing: **11.12.2012**

(86) International application number:
**PCT/NL2012/050879**

(87) International publication number:
**WO 2013/095111 (27.06.2013 Gazette 2013/26)**

(54) **SIMETHICONE FORMULATION**

SIMETHICONFORMULIERUNG

FORMULATION DE SIMÉTHICONE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.12.2011 EP 11193452**

(43) Date of publication of application:
**22.10.2014 Bulletin 2014/43**

(73) Proprietor: **Disphar International B.V.**
**7255 PZ Hengelo (NL)**

(72) Inventors:
• **VELADA, José Luis**
**NL-3826 BA Amersfoort (NL)**
• **DOSHI, Hiteshkumar Anilkant**
**Mumbai 4000080 (IN)**
• **TENDULKAR, Anjali Rajesh**
**Mumbai 400080 (IN)**
• **SHINDE, Vicky**
**Mumbai 400080 (IN)**

(74) Representative: **Swarte, Veronica Regina et al**
**Disphar International B.V.**
**Tolweg 15**
**3741 LM Baarn (NL)**

(56) References cited:
**WO-A1-2007/057924      WO-A1-2008/056200**
**US-A- 6 103 260**

**Description**

FIELD OF THE INVENTION

The present invention is in the field of pharmaceutical formulations comprising simethicone.

BACKGROUND OF THE INVENTION

Polydimethylsiloxanes are globally recognized both for their proven biocompatibility as well as for being one of the most tested materials for their safety. Simethicone is a mixture of poly(dimethylsiloxane) with silicon dioxide. It is known in pharmaceutical formulations as an active. The clinical use of simethicone is based on its antifoam properties, in particular it is used in the symptomatic treatment of flatulence, functional gastric bloating, and postoperative gas pains.
More often however, simethicone is used as an excipient. As excipients, many of the unique properties of polydimethylsiloxanes have been exploited in controlled release drug delivery systems due to their chemical stability, high level of purity, ease of use to manufacture different designs and very high permeability to active drugs.
Solid formulations of simethicone are well known. They are usually prepared by contacting simethicone liquid with solid carriers by various mixing techniques known in the art. The mixture is then converted to desired dosage forms. For economic manufacture and achieving the desired physical characteristics, the ingredients of the dosage form and the process conditions of manufacture need to be carefully selected. For large scale manufacture it is important to have sufficient flowability of the mixtures. Further, high compressibitily is desired to withstand the process of tabletting.
WO 2008/056200 concerns pharmaceutical compositions comprising simethicone and calcium silicate and a further absorber such as calcium phosphate. Also mannitol is mentioned as absorber. Of a number of ingredients including calcium phosphate and mannitol it is mentioned that these can be used as the sole absorbant for simethicone.
US 6,103,260 discloses oral solid dosage form preparations formed from a free flowing granular composition comprising simethicone and granular anhydrous tribasic calcium phosphate and/or dibasic calcium phosphate which is suitable for compression into a solid dosage form for oral administration. In this document, mannitol is suggested to be included in minor amounts as diluent or flavouring agent.
US 6,793,934 discloses an solid oral dosage form wherein a single solid carrier such as magnesium aluminosilicate, or granulated dibasic calcium phosphate is used to absorb simethicone.
When granulated carriers are used there is decreased surface area of contact between the liquid and the solid, which is not desirable to obtain a homogeneous distribution of the liquid in the carrier and to achieve larger absorption of simethicone in the carrier.
US 7,691,409 discloses a simethicone dosage form comprising silicified microcrystalline cellulose and magnesium aluminosilicate. Siliceous carriers are not desirable for simethicone formulations since such carriers could affect siliceous content of the liquid, thereby adversely affecting its activity. This is because, optimal activity of simethicone is not achieved beyond certain specified siliceous content.
WO 2007/057924 concerns laxative compositions comprising a plant extract as active ingredient combined with a saccharide that can be mannitol. In one example calcium phosphate tribasic is included in a minor amount.
There is, scope for development of free flowing and compressible formulations of simethicone, containing non siliceous carriers, that are convertible to desired pharmaceutical dosage forms.

SUMMARY OF THE INVENTION

The present inventors surprisingly found that a combination of calcium phosphate powder, simethicone and mannitol enables excellent flow characteristics as well as good compressibility. It was found that the composition according to the invention has flow characteristics and compressibility superior to compositions containing mannitol as the sole carrier. Further, the composition of the invention has higher flowability than a composition containing simethicone and calcium phosphate powder, without mannitol. Surprisingly, it was found that calcium phosphate powder does not flow, in the presence of mannitol, without simethicone. This is surprising because simethicone, being a viscous liquid is expected to retard the flow rather than assist the flow of solid mixtures. It is noted that the composition of the invention possess superior flow characteristics, as evidenced by the values of angle of repose, as compared to conventional formulations that contain other carriers such as calcium silicate (either individually or in combination with mannitol) or granular calcium phosphate. Additionally, the composition of the invention enables a nearly pH independent release in the pH range of 1 to 7.0. Further, it is noted that unlike the commercially available tablets, the tablets prepared from the composition of the invention maintains high stability with respect to disintegration time under stress conditions. Therefore, in the composition of the invention, a surprisingly effective synergy appears to operate among calcium phosphate powder, mannitol and simethicone.

DETAILED DESCRIPTION OF THE INVENTION

Accordingly, the invention provides a pharmaceutical composition comprising a mixture of simethicone, calcium phosphate powder and mannitol according to claims 1-9. In one embodiment, the invention provides a pharmaceutical composition comprising a mixture of simethicone, calcium phosphate powder and mannitol wherein the weight ratio mannitol to calcium phosphate powder is 1 : 1 or more than 1:1.
In another embodiment, the invention provides a pharmaceutical composition comprising a mixture of simethicone, calcium phosphate powder and mannitol wherein the weight ratio of mannitol to calcium phosphate powder is in the range of 1 : 1 to 7 : 1. In a further embodiment, the invention provides a pharmaceutical composition comprising a mixture of simethicone, calcium phosphate powder and mannitol wherein the weight ratio of mannitol to calcium phosphate powder is preferably in the range of 1: 1 to 4: 1, more preferably in the range of 1: 1 to 3: 1, even more preferably in the range of 1: 1 to 2: 1, even more preferably in the range of 1 : 1 to 1.5 : 1.

In another embodiment, the invention provides a pharmaceutical composition comprising a mixture of simethicone, calcium phosphate powder and mannitol wherein the weight ratio of simethicone to calcium phosphate powder is in the range of 1 : 0.6 to 1 : 2.2. In another embodiment, the invention provides a pharmaceutical composition comprising a mixture of simethicone, calcium phosphate powder and mannitol wherein the weight ratio of simethicone to calcium phosphate powder is in the range of 1 : 0.6 to 2.4, more preferably in the range of 1 : 0.8 to 1 : 2.2, more preferably in the range of 1 : 1 to 1 : 2.2, even more preferably in the range of 1 : 1.5 to 1 : 2.2.

In another embodiment, the invention provides a pharmaceutical composition comprising a mixture of simethicone, calcium phosphate powder and mannitol and an active ingredient other than simethicone. Preferably the active ingredient other than simethicone is selected from the group consisting of loperamide, olanzapine, risperidone, loratadine, hydrochlorothiazide, donepezil hydrochloride, ondansetron, clonazepam, clozapine, mitrazapine, oxcarbazapine, tramadol, cetirizine, lamotrizine, alprazolam, rizatriptan, zolmitriptan, montelukast, desloratadine and paracetamol.

In a further embodiment, the invention provides a pharmaceutical composition comprising a mixture of simethicone, calcium phosphate powder and mannitol and loperamide as an active ingredient.

In still a further embodiment, the invention provides a pharmaceutical composition comprising a mixture of simethicone, calcium phosphate powder and mannitol and loperamide as an active ingredient wherein more than 80% of loperamide is released at 30 minutes at a temperature of 37 °C and a stirring speed of 75 rpm in an aqueous solution having a pH in the range of 1 to 7.0.

The composition of the invention comprises simethicone, calcium phosphate powder and mannitol particles. Functionally, calcium phosphate powder and mannitol act as carriers for simethicone liquid. The calcium phosphate powder used in the composition of the invention may be dibasic or tribasic calcium phosphate powder, in their anhydrous or hydrated forms. Advantageously, the calcium phosphate powder may be calcium phosphate powder commercially available as E341(iii) (Sudeep Pharma), Tri-Cafos S® (Fabrik Budenheim), Calipharm T® and Calipharm A® (all from Innophos, USA). More advantageously, the calcium phosphate powder is a tribasic calcium phosphate powder.

Preferably, the composition of the invention comprises 10 to 60% by weight simethicone, 20 to 70% by weight of calcium phosphate powder and 20 to 70% by weight of mannitol. It is noted the weight percentages recited here are with respect to the total weight of the pharmaceutical composition. Advantageously, the composition of the invention comprises 10 to 20 % by weight of simethicone, 30 to 70% by weight, preferably 30 to 60% by weight of calcium phosphate powder and 30 to 70 % by weight, preferably 30 to 60% by weight of mannitol. In another embodiment, the composition of the invention comprises 10 to 20% by weight simethicone, 20 to 50% by weight of calcium phosphate powder and 20 to 50% by weight of mannitol with respect to the total weight of the composition. In yet another embodiment, the composition of the invention comprises 10 to 20% by weight simethicone, 20 to 40% by weight of calcium phosphate powder and 30 to 50% by weight of mannitol with respect to the total weight of the composition. In yet another embodiment, the composition of the invention comprises 10 to 20% by weight simethicone, 25 to 35% by weight of calcium phosphate powder and 30 to 40% by weight of mannitol with respect to the total weight of the composition.

As mentioned above, in one embodiment the present invention provides a pharmaceutical composition that comprises a mixture of simethicone, calcium phosphate powder and mannitol. Preferably the pharmaceutical composition comprises 50 to 100 % by weight, more preferably 55 to 95 % by weight, still more preferably 60 to 80% by weight of the mixture of simethicone, calcium phosphate powder and mannitol.

In a further preferred embodiment, the mixture of simethicone, calcium phosphate powder and mannitol that is comprised in the pharmaceutical composition comprises 10 to 70% by weight simethicone, 20 to 80% by weight of calcium phosphate powder and 20 to 80% by weight of mannitol with respect to the mixture. It is noted that the percentages recited here are with respect to the total weight of the mixture of simethicone, calcium phosphate powder and mannitol that is comprised in the pharmaceutical composition. More preferably, the mixture of simethicone, calcium phosphate powder and mannitol comprises 10 to 60% by weight of simethicone, more preferably 10 to 50%, more preferably 10 to 40%, more preferably 10 to 30% and still more preferably 10 to 20% by weight of simethicone with respect to the mixture, 20 to 70% by weight of calcium phosphate powder, more preferably 20 to 60%, more preferably 20 to 50% and still more preferably 20 to 40% by weight of calcium phosphate powder with respect to the mixture and 20 to 70% by weight of mannitol, more preferably 20 to 60%, more preferably 20 to 50%, more preferably 30 to 50%, more preferably 40 to 50% by weight of mannitol with respect to the mixture.

In one embodiment the invention provides a pharmaceutical composition that comprises 55 to 95 % by weight of a mixture of simethicone, calcium phosphate powder, wherein the mixture of simethicone, calcium phosphate powder and mannitol comprises 10 to 30%, more preferably 10 to 20% by weight of simethicone with respect to the mixture, 20 to 50% more preferably 20 to 40% by weight of calcium phosphate powder with respect to the mixture and 30 to 50%, more preferably 40 to 50% by weight of mannitol with respect to the mixture. In this embodiment, preferably the pharmaceutical composition comprises 10 to 20% by weight simethicone, 20 to 40% by weight of calcium phosphate powder and 30 to 50% by weight of mannitol.

Mannitol used in the composition of the invention may be mannitol that is commercially available from manufacturer Roquette, France, under different brand names like Pearlitol SD 100® Pearlitol SD 200®, Pearlitol SD 300®, Pearlitol 160DC®, Pearlitol 200 DC®, Pearlitol 300 DC®, Pearlitol 400 DC®, Pearlitol 500DC® and mannitol available from Pharmatrans Sanaq AG under the trade name M-cell®.

Simethicone used in preparing the composition of the invention may be simethicone commercially available under the trade names Sentry Simethicone® (Nusil Technologies) BC Antifoam C100® (Basildon Chemicals), Filix 110® (Riocare) and Q7-2243 LVA® simethicone (Dow Corning)

The calcium phosphate that is used in preparing the composition of the invention is in the form of a powder. In the context of this invention "powder" is to be read as opposed to "granular". So in one embodiment according to the invention, "calcium phosphate powder" has the meaning of "non-granular calcium phosphate" i.e, calcium phosphate powder devoid of granular calcium phosphate. Calcium phosphate powder or granular calcium phosphate are terms commonly used in the art and well known to the skilled person.. In one embodiment according to the invention, "calcium phosphate powder" means particles of calcium phosphate having particle size less than 50 microns. In a preferred embodiment "calcium phosphate powder" means that more than 95% of the calcium phosphate powder passes through a sieve of 80 mesh. In one embodiment according to the invention the calcium phosphate comprised in the pharmaceutical composition comprising a mixture of simethicone, calcium phosphate powder and mannitol, is solely in powder form, or, in other words, preferably the mixture of simethicone, calcium phosphate powder and mannitol does not comprise granular calcium phosphate. In a preferred embodiment, the pharmaceutical composition according to the invention does not comprise granular calcium phosphate, or in other words, granular calcium phosphate is excluded from the composition according to the invention.

The composition of the invention can include further pharmaceutically acceptable excipients. The pharmaceutically acceptable excipients that can be contained in the composition of the invention include, but are not limited to, binders such as starches microcrystalline cellulose; celluloses such as hydroxypropyl cellulose, hydroxyethyl cellulose, hydroxypropylmethyl cellulose (HPMC), ethyl cellulose, sodium carboxy methyl cellulose; natural gums like acacia, alginic acid, guar gum; liquid glucose, dextrin, povidone, syrup, polyethylene oxide, polyvinyl pyrrolidone, poly-N-vinyl amide, polyethylene glycol, gelatin, polypropylene glycol, tragacanth, combinations thereof and other materials known to one of ordinary skill in the art and mixtures thereof, fillers or diluents including, but not limited to, confectioner's sugar, compressible sugar, dextrates, dextrin, dextrose, fructose, lactitol, mannitol, sucrose, starch, lactose, xylitol, sorbitol, talc, microcrystalline cellulose, calcium carbonate, calcium sulphate, and the like can be used, lubricants selected from, but not limited to, those conventionally known in the art such as magnesium stearate, aluminium stearate or calcium stearate or zinc stearate, polyethylene glycol, glyceryl behenate, mineral oil, sodium stearyl fumarate, stearic acid, hydrogenated vegetable oil and talc, glidants including, but not limited to powdered cellulose, starch and other materials known to one of ordinary skill in the art, surface-active agent such as dioctyl sodium sulfosuccinate (DSS), triethanolamine, sodium lauryl sulphate (SLS), polyoxyethylene sorbitan and poloxalkol derivatives, quaternary ammonium salts or other pharmaceutically acceptable surface-active agents known to one ordinary skilled in the art. It is not excluded that the pharmaceutical composition may contain calcium phosphate, either in granular or powdered form and/or mannitol as excipients, thus not included in the mixture with simethicone. In this case calcium phosphate and/or mannitol have a different function than acting as carrier for simethicone. In one embodiment the composition according to the invention does not comprise calcium silicate.

The mixture in the composition of the invention has desirable values of angle of repose and/or compressibility required for large scale manufacture of solid dosage forms. The mixture in the composition of the invention has compressibility values in the range of 10 to 25% or angle of repose values in the range of 20 to 35 degrees or has values of compressibility and angle of repose in both these ranges.

The composition of the invention can be in the form of a powder, granules, pellets or tablets. Advantageously, the mixture in the composition of the invention is a free flowing mixture of powdered carriers with liquids, e.g. powdered material carrying simethicone liquid, that have sufficiently high compressibility required to be shape formed as desired. Further, the composition of the invention displays an immediate release profile at varied ranges of pH and also retains this profile upon storage.

Advantageously, the mixture, i.e. the mixture of simethicone, calcium phosphate powder and mannitol, in the composition of the invention consists essentially of simethicone, calcium phosphate powder and mannitol.

The composition of the invention is prepared by contacting calcium phosphate powder and mannitol with simethicone liquid to form a solid-liquid blend. The solid and the liquid are usually contacted in a rapid mixer granulator (RMG) resulting in a lubricated blend. Compression of the blend may be carried out by a suitable compression machine. Typically, a compression machine with 'D' tooling using appropriate punches is used.

Throughout the specification, the angle of repose is a quantitative indication for the flowability and can be measured by using a cone formation method and compressibility was determined by using bulk density and tapped density values. Angle of repose was determined using a funnel mounted on a stand at specific height. The funnel mouth was closed using a stopper. The powder of which angle of repose was to be determined was filled in funnel and funnel stopper was removed. The diameter of the hip formed was measured using a calibrated Vernier caliper. The angle of repose was then calculated using following formula:

$$\tan(\alpha) = \text{height of hip}/0.5 \times \text{diameter of hip}.$$

To determine the compressibility, firstly the bulk density ($V_1$) of powder was determined using a calibrated measuring cylinder (100 ml). The tapped density ($V_2$) was determined after tapping the cylinder until no further change was seen in tapped volume (No. of Taps: 1250). Compressibility was then calculated using following formula:

$$\text{Compressibility} = 100 \times (V_1 - V_2/V_1).$$

Hardness of the tablets was determined by measuring resistance to crushing. The measurements were carried out using Eureka hardness tester. Disintegration time was calculated using a ED-2AL disintegration tester from Electrolab®.

Dissolution studies for determining release of the active ingredient at various pHs were carried out using a TDT-08 L dissolution apparatus from Electrolab®

The advantageous flow characteristics and good compressibility exhibited by the composition of the invention enables facile processing of the composition into various dosage forms. The composition of the invention not containing a siliceous carrier was capable of incorporating high quantities of simethicone. The superior utilization and distribution of simethicone by the carrier particles ensures reduced cost and efficiency of manufacture. The high values of release of the second active ingredient in the composition of the invention indicates that the release profile of the second active ingredient is substantially independent of pH in the acidic to neutral pH range. It also indicates that the release of the second active ingredient in the composition is not retarded by the other active ingredient, namely simethicone. The substantially pH independent release profile of active ingredient for the compositions of the invention in the pH range of 1 to 7 enables consistency of release of the active ingredient in the gastrointestinal tract. Further, the substantial retention of disintegration profile demonstrated by the composition of the invention ensures sustained pharmaceutical efficiency of the composition upon storage. Furthermore, the process of manufacture of the composition of the invention allows scalability.

Additionally, as seen before, the composition of the invention enables a nearly pH independent release in the pH range of 1 to 7.0. Such pH independent release is both advantageous and all the more surprising because, besides the commercially available tablets (Imodium® Plus), even the API (loperamide hydrochloride) exhibit *pH dependent* dissolution characteristics in the pH range of 1 to 7. Further, it may also be noted that unlike the commercially available tablets, the tablets prepared from the composition of the invention maintains high stability with respect to disintegration time under stress conditions. Therefore, in the composition of the invention, a surprisingly effective synergy appear to operate among calcium phosphate powder, mannitol and simethicone. Still further, the composition of the invention possess efficient distribution of liquid in the carrier.

EXAMPLES

**Example 1:** *Comparative study of compressibility and flowability of simethicone compositions prepared by using various carriers at different relative proportions*

The absorption of simethicone over various carriers was compared, in different experiments, at their different relative proportions and quantities displayed in table 1.

Table 1 displays results of comparative study of compressibility and flowability of simethicone compositions prepared by using various carriers at different relative proportions.

The compositions were prepared as follows:

320 mg/tablet of first carrier (displayed in column 2 of table I) and corresponding amount of second carrier (displayed in column 3 of table I) in the relative proportions as displayed in column 5 of table I and sifted through 40 mesh ASTM screen using mechanical sifter were dry mixed in a 1.0 L bowl of RMG at an impeller speed of 150 rpm. 130 mg/tablet of simethicone was added through the charging port under mixing at 150 rpm. The addition rate was maintained such that the simethicone addition was completed in 5 - 7 minutes. The simethicone container was rinsed with the adsorbate from RMG & the rinsings were added to the bowl of RMG & mixed for one minute to form the lubricated blend. The lubricated blend was compressed with a suitable compression machine with 'D' tooling using appropriate punches.

The various carriers studied were mannitol commercially available with the trade names Pearlitol SD 100®, Pearlitol SD 200®, Pearlitol SD 300® (All from Roquette, France) and M-cell® (Pharmatrans Sanaq AG), calcium phosphate commercially available as A-Tab®, Calipharm A® (Innophos USA), E 341(iii) (Sudeep pharma) and calcium silicate commercially available under the trade name Hubersorb 250 NF® (Huber Engineered materials). The amount of carriers displayed in table 1 are in weight per tablet. For comparison, experiments were also performed with single carriers (Experiment Nos.1, 2, 5, 10, 12) or without simethicone (Experiment Nos. 8,9). In all the experiments, the total amount of the carrier (in mg/tablet) was maintained at 566.23.

From table 1 it is clear that the composition of the invention has flow characteristics and compressibility superior to compositions containing mannitol as the sole carrier. Further, the composition of the invention has higher flowability than a composition containing simethicone and calcium phosphate powder , without mannitol. It is also clear from table 1 that a combination of calcium phosphate powder, simethicone and mannitol enables excellent flow characteristics as well as good compressibility.

Surprisingly, calcium phosphate powder does not flow, in the presence of mannitol, without simethicone. This is surprising because simethicone, being a viscous liquid is expected to retard the flow rather than assist the flow of solid mixtures. It is noteworthy that the composition of the invention possess superior flow characteristics, as evidenced by the values of angle of repose, as compared to conventional formulations that contain other carriers such as calcium silicate (either individually or in combination with mannitol) or granular calcium phosphate.

**Table 1:** Comparative study of compressibility and flowability of simethicone compositions prepared by using various carriers at different relative proportions

| Ex pt No | First carrier | Second carrier | Amount of simethicone (mg/tablet) | Second carrier: first carrier weight ratio | Angle of repose (Degrees) | Compres sibility (%) |
|---|---|---|---|---|---|---|
| 1 | Calcium phosphate granular (A-Tab®) | Nil | 130 | 0:1† | 31.98 | 23.077 |
| 2 | Calcium phosphate powder (Calipharm A®) | Nil | 130 | 0:1† | No flow | 38.26 |
| 3 | Calcium phosphate powder (E-341 (iii)) | Mannitol (Pearlitol SD 200®) | 130 | 1:1 | 30.29 | 24.138 |
| 4 | Calcium phosphate powder (E-341 (iii) ) | Mannitol (M-Cell®) | 130 | 1:1 | 26.3 | 24.31 |
| 5 | Calcium phosphate powder (E-341 (iii) ) | Nil | 130 | 0:1 | No flow | 41.22 |
| 6 | Calcium phosphate powder (E-341 (iii) ) | Mannitol (Pearlitol SD 100®) | 130 | 1:1 | 29.84 | 23.68 |
| 7 | Calcium phosphate powder (E-341 (iii)) | Mannitol (Pearlitol SD 300) | 130 | 1:1 | 29.86 | 27.50 |

(continued)

| Ex pt No | First carrier | Second carrier | Amount of simethicone (mg/tablet) | Second carrier: first carrier weight ratio | Angle of repose (Degrees) | Compres sibility (%) |
|---|---|---|---|---|---|---|
| 8 | Calcium phosphate powder (E-341 (iii)) | Mannitol (Pearlitol SD 2000) | 0 | 1:1 | No flow | 41.379 |
| 9 | Calcium phosphate powder (E-341 (iii)) | Mannitol (M-Cell®) | 0 | 1:1 | No flow | 36.29 |
| 10 | Nil | Mannitol (Pearlitol SD 200®) | 130 | 1 : 0†† | No flow | 37.21 |
| 11 | Calcium silicate Hubersorb® 250 NF | Mannitol (Pearlitol SD 200®) | 130 | 1:1 | No flow | 33.33 |
| 12 | Calcium silicate Hubersorb® 250 NF | Nil | 130 | 0:1††† | No flow | 39.22 |

In these experiments 566.23 mg of granular/powdered calcium phosphate was utilized to absorb 130 mg of simethicone in the absence of mannitol.

†† In this experiment, 566.23 mg of mannitol was utilized to absorb 130 mg of simethicone, in the absence of any other carrier.

††† In this experiment, 566.23 mg of calcium silicate was utilized to absorb 130 mg of simethicone, in the absence of any other carrier.

The angle of repose and compressibility values of the composition of the invention studied as a function of mannitol to calcium phosphate powder ratio (Experiment No's 3 to 6) as displayed against the corresponding values of the composition having mannitol: calcium phosphate powder ratio of 0.25:1 and 0.50:1 is given in table 2.

**Table 2:** Comparative analysis of formulation parameters with respect to variation relative quantities of calcium phosphate powder and mannitol

| Expt No | Mannitol : Calcium phosphate powder ratio | Simethicone: Calcium phosphate powder weight ratio | Angle of repose (Degrees) | Compressibility (%) |
|---|---|---|---|---|
| 1 | 0.25:1 | 1:3.8 | No flow | 37.04 |
| 2 | 0.50:1 | 1:3.3 | No flow | 40.74 |
| 3 | 1:1 | 1:2.2 | 30.29 | 24.14 |
| 4 | 3:1 | 1:1.4 | 29.86 | 19.23 |
| 5 | 5:1 | 1:0.9 | 27.52 | 13.04 |
| 6 | 7:1 | 1:0.6 | 29.22 | 25 |

From Table 2 it is evident that, advantageously, the composition of the invention has a mannitol: Calcium phosphate powder ratio of at least 1:1. The amounts of mannitol at a mannitol: Calcium phosphate powder ratio of less than 1:1 are found to impart negligible flow and unacceptable values of compressibility. It may be noted that for the composition of the invention having mannitol : Calcium phosphate powder ratio of at least 1:1, a simethicone: Calcium phosphate powder ratio of at least 1: 2.2 is obtained. Such a ratio signifies high absorption of simethicone in the carrier

**Example 2** *Process of preparing loperamide-simethicone tablets*

Loperamide hydrochloride granules obtained by wet granulation of 2 mg/tablet of loperamide HCl powder, 100 mg/tablet of microcrystalline cellulose and 37 mg/tablet of sodium starch glycollate, all sifted through 40 mesh ASTM, were mixed in a contablender for 10 minutes with the lubricated blend obtained as in Example 1. A mannitol: calcium phosphate powder weight ratio of 1.2:1 and simethicone: Calcium phosphate powder weight ratio of 1:2 was maintained. The mixture so obtained was compressed with a suitable compression machine with 'D' tooling to obtain 16.6 X 6.8 mm sized capsule shaped tablets.

The above-mentioned tablets were studied for release profile, at 30 minutes, in solutions of varied pH such as 1.2, 4.5 and 6.8 respectively in aqueous solutions of 0.1 N HCl, acetate buffer and phosphate buffer. Comparison was made with loperamide-simethicone caplets commercially available under the trade name, Imodium® Plus. It was found that 92%, 52% and 20% of loperamide was released from the Imodium® Plus tablets at a pH of 1.2, 4.5 and 6.8 respectively while from the tablets of the invention prepared as above, 94%, 95% and 82 % of loperamide was released at a pH of 1.2, 4.5 and 6.8 respectively. In other words, in the pH range of 1 to 7, more than 80% of the release has been observed, for the tablets of the invention. At the same time, in the same pH range, the release profile of Imodium® Plus tablets has been found to be pH dependent. This indicates that the tablet composition of the present invention allows a nearly pH independent release as compared to the Imodium® Plus tablets. Moreover, such a release pattern indicates that the release of active ingredient of the tablet of the invention is not influenced by simethicone, the other active ingredient in the composition. The disintegration time of the tablets of the invention is compared with Imodium® Plus tablets in the table 3 below.

**Table 3:** Disintegration time

| Nature of tablet | Pristine (Untreated) | 40°C/3M | 60°C/1M | $\Delta t^{\dagger}_{0-40}$ | $\Delta t^{\dagger\dagger}_{0-60}$ |
|---|---|---|---|---|---|
| Imodium® Plus tablet | 6 minutes, 40 seconds | 8 minutes, 57 seconds | 30 minutes | 2 minutes, 17 seconds | 23 minutes, 20 seconds |
| Tablets prepared as in example 2 | 3 minutes | 4 minutes, 25 seconds | 8 minutes, 24 seconds | 1 minutes, 25 seconds | 5 minutes, 24 seconds |

$\dagger$ Difference in disintegration time of tablets in the untreated state and the tablets after being subjected to accelerated disintegration at 40°C for 1 month.
$\dagger\dagger$ Difference in disintegration time of tablets after being subjected to accelerated disintegration at 60 degrees for 1 month.

As evident from the above table, the disintegration time of Imodium® Plus tablets increased by 23 minutes and 20 seconds while the tablets of the invention showed only a relatively less increase, of 5 minutes and 24 seconds, in the disintegration time, after being subjected to accelerated disintegration at 60 degrees for 1 month.

Similarly, after being subjected to accelerated disintegration at 40 degrees for 3 months, it was found that the disintegration time of Imodium® Plus tablets increased by 2 minutes and 17 seconds while the tablets of the invention showed a less remarkable increase of 1 minutes and 25 seconds in the disintegration time.

From this, it is clear that the tablets of the invention enables retention of disintegration time over stress conditions/ shelf life period.

**Claims**

1. A pharmaceutical composition comprising a mixture of simethicone, calcium phosphate powder and mannitol and wherein simethicone is present in 10 to 60% by weight, calcium phosphate powder is present in 20 to 70% by weight and mannitol is present in 20 to 70% by weight of the composition, and wherein the weight ratio of mannitol to calcium phosphate powder is in the range of 1 : 1 to 7 : 1, and wherein the weight ratio of simethicone to calcium phosphate powder is in the range of 1 : 0.6 to 1 : 2.2.

2. The composition according to claim 1 wherein the mixture of simethicone, calcium phosphate powder and mannitol does not comprise granular calcium phosphate.

3. The composition according to claim 1 or 2 wherein the mixture essentially consists of simethicone, calcium phosphate powder and mannitol.

4. The composition according to any one of claims 1 to 3 which comprises 10 to 20% by weight simethicone, 20 to 50% by weight of calcium phosphate powder and 20 to 50% by weight of mannitol.

5. The composition according to any one of claims 1 to 4 further comprising an active ingredient selected from the group consisting of loperamide, olanzapine, risperidone, loratadine, hydrochlorothiazide, donepezil hydrochloride, ondansetron, clonazepam, clozapine, mitrazapine, oxcarbazapine, tramadol, cetrizine, lamotrizine, alprazolam, rizatriptan, zolmitriptan, montelukast, desloratadine and paracetamol.

6. The composition according to claim 5 wherein the active ingredient is loperamide.

7. The composition according to claim 6 wherein more than 80% of the active ingredient is released at 30 minutes at a temperature of 37°C and a stirring speed of 75 rpm in an aqueous solution having pH in the range of 1 to 7.0.

8. A pharmaceutical composition comprising 55 to 95 % by weight of a mixture of simethicone, calcium phosphate powder, and mannitol, wherein the mixture of simethicone, calcium phosphate powder and mannitol comprises 10 to 30% by weight of simethicone with respect to the mixture, 20 to 50% by weight of calcium phosphate powder with respect to the mixture and 30 to 50% by weight of mannitol with respect to the mixture.

9. The pharmaceutical composition according to claim 8 , comprising 10 to 20% by weight simethicone, 20 to 40% by weight of calcium phosphate powder and 30 to 50% by weight of mannitol, based on total weight of the pharmaceutical composition.


**Patentansprüche**

1. Pharmazeutische Zusammensetzung, die eine Mischung aus Simethicon, Calciumphosphatpulver und Mannit umfasst, worin Simethicon mit 10 - 60 Gewichtsprozent zugegen ist, Calciumphosphatpulver mit 20 - 70 Gewichtsprozent zugegen ist und Mannit mit 20 - 70 Gewichtsprozent der Zusammensetzung zugegen ist, und worin das Gewichtsverhältnis von Mannit zu Calciumphosphatpulver im Bereich von 1:1 bis 7:1 ist und worin das Gewichtsverhältnis von Simethicon zu Calciumphosphatpulver im Bereich von 1:0,6 zu 1:2,2 ist.

2. Zusammensetzung gemäß Anspruch 1, worin die Mischung aus Simethicon, Calciumphosphatpulver und Mannit kein granulares Calciumphosphat umfasst.

3. Zusammensetzung gemäß Anspruch 1 oder 2, worin die Mischung im Wesentlichen aus Simethicon, Calciumphosphatpulver und Mannit besteht.

4. Zusammensetzung gemäß einem der Ansprüche 1 bis 3, die 10 - 20 Gewichtsprozent Simethicon, 20 - 50 Gewichtsprozent Calciumphosphatpulver und 20 - 50 Gewichtsprozent Mannit umfasst.

5. Zusammensetzung gemäß einem der Ansprüche 1 bis 4, die ferner einen Wirkstoff umfasst, der aus der Gruppe ausgewählt ist, die aus Loperamid, Olanzapin, Risperidon, Loratadin, Hydrochlorothiazid, Donepezilhydrochlorid, Ondansetron, Clonazepam, Clozapin, Mitrazapin, Oxcarbazapin, Tramadol, Cetirizin, Lamotrizin, Alprazolam, Rizatriptan, Zolmitriptan, Montelukast, Desloratidin und Paracetamol besteht.

6. Zusammensetzung gemäß Anspruch 5, worin der Wirkstoff Loperamid ist.

7. Zusammensetzung gemäß Anspruch 6, worin mehr als 80 % des Wirkstoffs nach 30 Minuten bei einer Temperatur von 37 °C und einer Rührgeschwindigkeit von 75 UpM in einer wässrigen Lösung mit einem pH im Bereich von 1 - 7,0 freigesetzt wird.

8. Pharmazeutische Zusammensetzung, die 55 - 95 Gewichtsprozent einer Mischung aus Simethicon, Calciumphosphatpulver und Mannit umfasst, worin die Mischung aus Simethicon, Calciumphosphatpulver und Mannit 10 - 30 Gewichtsprozent Simethicon in Bezug auf die Mischung, 20 - 50 Gewichtsprozent Calciumphosphatpulver in Bezug auf die Mischung und 30 - 50 Gewichtsprozent Mannit in Bezug auf die Mischung umfasst.

9. Pharmazeutische Zusammensetzung gemäß Anspruch 8, die 10 - 20 Gewichtsprozent Simethicon, 20 - 40 Gewichtsprozent Calciumphosphatpulver und 30 - 50 Gewichtsprozent Mannit umfasst, bezogen auf das Gesamtgewicht der pharmazeutischen Zusammensetzung.

**Revendications**

1. Composition pharmaceutique comprenant un mélange de simethicone, de poudre de phosphate de calcium et de mannitol, dans laquelle le simethicone est présent de 10 à 60% en poids, la poudre de phosphate de calcium est présente de 20 à 70% en poids et le mannitol est présent de 20 à 70% en poids de la composition, et dans laquelle le ratio en poids du mannitol à la poudre de phosphate de calcium est dans la gamme de 1 : 1 à 7 : 1, et dans laquelle le ratio en poids du simethicone à la poudre de phosphate de calcium est dans la gamme de 1 : 0.6 à 1 : 2.2.

2. Composition selon la revendication 1 dans laquelle le mélange de simethicone, de poudre de phosphate de calcium et de mannitol ne comprend pas de phosphate de calcium granulaire.

3. Composition selon la revendication 1 ou 2 dans laquelle le mélange consiste essentiellement en simethicone, poudre de phosphate de calcium et mannitol.

4. Composition selon l'une quelconque des revendications 1 à 3 laquelle comprend 10 à 20% en poids de simethicone, 20 à 50% en poids de poudre de phosphate de calcium et 20 à 50% en poids de mannitol.

5. Composition selon l'une quelconque des revendications 1 à 4 comprenant en outre un ingrédient actif choisi parmi le groupe constitué par loperamide, olanzapine, risperidone, loratadine, hydrochlorothiazide, donepezil hydrochloride, ondansetron, clonazepam, clozapine, mitrazapine, oxcarbazapine, tramadol, cetrizine, lamotrizine, alprazolam, rizatriptan, zolmitriptan, montelukast, desloratadine et paracetamol.

6. Composition selon la revendication 5 dans laquelle l'ingrédient actif est loperamide.

7. Composition selon la revendication 6, dans laquelle plus de 80% de l'ingrédient actif est libéré à 30 minutes à une température de 37°C et une vitesse d'agitation de 75 rpm dans une solution aqueuse ayant un pH dans la gamme de 1 à 7.0

8. Composition pharmaceutique comprenant 55 à 95% en poids d'un mélange de simethicone, de poudre de phosphate de calcium et de mannitol, dans laquelle le mélange de simethicone, poudre de phosphate de calcium et de mannitol comprend 10 à 30 % en poids de simethicone par rapport au mélange, 20 à 50 % en poids de poudre de phosphate de calcium par rapport au mélange et 30 à 50% en poids de mannitol par rapport au mélange.

9. Composition pharmaceutique selon la revendication 8 comprenant 10 à 20% en poids de simethicone, 20 à 40 % en poids de poudre phosphate de calcium et 30 à 50% en poids de mannitol, basé sur le poids total de la composition pharmaceutique.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008056200 A
- US 6103260 A
- US 6793934 B
- US 7691409 B
- WO 2007057924 A